(19) Europäisches Patentamt
European Patent Office
Office européen des brevets

(11) EP 2 488 148 B1

(12) **EUROPEAN PATENT SPECIFICATION**

(45) Date of publication and mention of the grant of the patent:
**26.04.2017 Bulletin 2017/17**

(21) Application number: **10771206.9**

(22) Date of filing: **12.10.2010**

(51) Int Cl.:
***A61K 8/04*** *(2006.01)* ***A61Q 17/04*** *(2006.01)*
***A61K 8/81*** *(2006.01)* ***C03C 4/08*** *(2006.01)*
***C08K 13/04*** *(2006.01)* ***C09D 7/12*** *(2006.01)*
***C09D 11/00*** *(2014.01)* ***D06M 23/12*** *(2006.01)*
***D21C 9/00*** *(2006.01)* ***D06M 11/79*** *(2006.01)*
***D06M 23/08*** *(2006.01)* ***C03C 17/00*** *(2006.01)*
***C08K 3/00*** *(2006.01)* ***A61K 8/02*** *(2006.01)*

(86) International application number:
**PCT/IB2010/054609**

(87) International publication number:
**WO 2011/045740 (21.04.2011 Gazette 2011/16)**

(54) **METHODS OF PHOTOPROTECTING A MATERIAL AGAINST SOLAR UV RADIATION USING PHOTONIC PARTICLES; COMPOSITIONS**

VERFAHREN ZUM SCHÜTZEN EINES MATERIALS GEGEN UV-STRAHLUNG MITTELS PHOTONENPARTIKEL, ZUSAMMENSETZUNGEN

PROCÉDÉS DE PHOTOPROTECTION D'UN MATÉRIAU CONTRE LES RAYONNEMENTS UV SOLAIRES AU MOYEN DE PARTICULES PHOTONIQUES; COMPOSITIONS CORRESPONDANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.10.2009 FR 0957134**
**29.10.2009 US 256152 P**
**17.02.2010 FR 1051141**
**19.02.2010 US 306319 P**

(43) Date of publication of application:
**22.08.2012 Bulletin 2012/34**

(73) Proprietor: **L'Oréal**
**75008 Paris (FR)**

(72) Inventors:
- **SIMONNET, Jean-Thierry**
**F-94230 Cachan (FR)**
- **LUCET-LEVANNIER, Karine**
**F-92500 Rueil-malmaison (FR)**

(74) Representative: **Nony**
**11 rue Saint-Georges**
**75009 Paris (FR)**

(56) References cited:
**WO-A1-2006/136719** **WO-A2-2006/116567**
**WO-A2-2006/136724** **US-A1- 2002 024 163**
**US-A1- 2006 002 875** **US-B2- 6 894 086**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 488 148 B1

## Description

The invention relates to compositions for use in a method of photoprotecting human keratinous material against solar UV radiation, using a dispersion of photonic particles.

## Prior art

Current photoprotective compositions use combinations of various screening agents, especially soluble or insoluble organic screens. The absorption spectrum of each of said screens is rarely broad enough to cover the whole UV spectrum, and combinations are necessary.

Further, a large number of soluble organic screens may cause compatibility problems with the ingredients usually contained in them, especially as a result of interactions with other organic screens or with active molecules such as antioxidants or vitamins, and their photostability may not be entirely satisfactory. Many patents are concerned with solving this problem, indicating that this problem is recurrent.

Many non-cosmetic industry sectors also use UV screens to photoprotect various materials against the effects of UV radiation, in particular solar radiation.

This applies in particular with paint, ink, or protective coating formulations for applying to substances that are permanently exposed to UV radiation, such as building materials, materials used in the automobile industry, or plastic packaging materials. In particular, UV screens are being developed for colorant formulations, which screens need to be transparent, photostable, compatible with the usual ingredients contained in said formulations, and effective in providing the color with the desired resistance to light.

This also applies with polymer compositions used in particular in the manufacture of plastics materials that are stable in storage; they need the development of UV light screens that are particularly adapted to methods of manufacturing and transforming polymers, and in particular they have to be able to tolerate the high temperatures used in extrusion.

In the natural fiber and/or artificial fiber and/or synthetic fiber industry, broad spectrum photostable UV screens are being developed that are compatible with methods of manufacturing said fibers, in particular in the context of the manufacture of polyamide fibers such as nylon, which filters are resistant to high temperatures and enable UV protection to be incorporated during extrusion. Further, UV screens are being developed that have good affinity, good adhesion to fibers, and thus in particular that provide good resistance to frequent washing. The UV screens being developed must also provide both good protection of the textile fibers and also of the skin and other human keratinous material in contact with said fibers.

The mineral or organic glass industry, in particular glass used in ophthalmology, is developing UV screens that are to have a broad spectrum (active in the UVA and in the UVB regions), and that are photostable, transparent, and compatible with the various techniques for treating glass such as methods of keying onto the glass matrix or applying a photoprotective coating, for example with polycarbonate glass.

Application WO 06/136724 shows that it is known to use monodisperse particles that are capable of forming a lattice and that have optical screening properties in the UVB, UVA and infrared. In that application, the particles have to become organized on the skin.

In the publication by J. Wang, Polym Int 57:509 - 514, 2008, the authors produced monodisperse PMMA spheres with various diameters (95 nm, 114 nm, 134 nm, 142 nm and 150 nm). Each diameter corresponded to a reflection maximum (250 nm, 280 nm, 330 nm, 350 nm, 380 nm) when the particles are organized into a lattice.

Application WO 08/007267 describes the use of hollow particles that are capable of becoming organized into a photonic crystal for makeup and UV photoprotection applications.

Patent US 6 894 086 describes various photonic particles, especially colored, or reflecting electromagnetic radiation outside the visible spectrum.

Applications US 2003/0116062 and US 2006/0002875 describe photonic particles, but do not use them in a method of photoprotection against solar ultraviolet radiation.

The publication by A. Stein (Chem Mater 2002, 14, 3305 - 3315) discloses photonic particles having a reflection band in the long UVA spectrum (374 nm).

The publication by E. Thomas (Nat Mat Vol 6, 957 - 960, 2008) discloses a gel having a reflection spectrum including a plurality of reflection peaks of distinct order.

There is a need to benefit from non-soluble screening materials that can be used to cover the UVA and/or UVB spectrum, that are completely harmless, inert towards the environment, photostable, and not photoreactive, and that do not have compatibility problems with the other constituents of the compositions containing them, do not modify the mechanical properties of the materials of the packaging in a negative manner, and do not release nanoparticles, and that are transparent to visible light.

There is also a need to develop novel materials that screen UV radiation and that are adapted to photoprotecting industrial materials such as those mentioned above.

The invention aims to achieve all or some of these objects.

In exemplary embodiments, the invention provides a composition for use in a method of photoprotecting human keratinous material against solar UV radiation as recited in claim 1.

The present description also discloses a method of photoprotecting a material against solar UV radiation, comprising treating said material by means of a composition comprising a dispersion of photonic particles with a mean size in the range 1 μm [micrometer] to 500 μm, each comprising a diffracting arrangement of monodisperse nanoparticles or voids, the diffraction spectrum of said arrangement including a first order reflection peak in the wavelength range 250 nm [nanometer] to 400 nm, or comprising integrating said dispersion of photonic particles into said material.

The present description also discloses a non-therapeutic, and in particular cosmetic, method of photoprotecting human keratinous material against solar UV radiation, comprising applying a cosmetic composition comprising a dispersion of photonic particles with a mean size in the range 1 μm to 500 μm, each comprising a diffracting arrangement of monodisperse nanoparticles or voids, the diffraction spectrum of said arrangement including a first order reflection peak in the wavelength range 250 nm to 400 nm.

In the context of the invention, the term "diffracting arrangement" means a set of particles or voids diffracting incident light in a manner that screens UV and/or produces coloration and/or modifies the spectral reflectance, depending on the application.

The presence of a first order reflection peak in the wavelength range 250 nm to 400 nm means that the arrangement diffracts light of at least one wavelength in the range 250 nm to 400 nm with an interference order equal to 1, thereby at least partially reflecting it.

Such a first order reflection peak in the UV implies that the reflection peaks of the following orders are located at shorter wavelengths, and thus outside the visible region. This renders the arrangement colorless and facilitates the production of a composition that is colorless, which is preferable in the context of a sunscreen application.

By way of example, the composition in accordance with the invention has an SPF index of at least 10, preferably 15, more preferably at least 30, 45 or 60. The SPF index (Sunscreen Protection Factor) is defined in the article "A new substrate to measure sunscreen protection factors throughout the ultraviolet spectrum", J. Soc. Cosmet. Chem., 40, 127 - 133 (May/June 1989).

The formulation of the composition is, for example, selected such that the composition has a transmission factor of 70%, 60%, 50%, 40%, 30%, 20%, 10%, 5% or less, or preferably of 1% or less, for at least one wavelength in the range 250 nm to 400 nm, preferably over the whole of said range. Screening is much better when the transmission factor is low in the range 250 nm to 400 nm.

The present description also discloses a method of preparing a cosmetic composition, comprising dispersing the photonic particles of the invention in a cosmetically acceptable medium.

This composition may present, unless the contrary is specified, all the properties of the cosmetic compositions of the invention described below.

In accordance with other exemplary embodiments, the invention provides a photonic particle with a mean size in the range 1 μm [micrometer] to 500 μm, especially in the range 1 μm to 300 μm, comprising a diffracting arrangement of hollow nanoparticles.

The present description also discloses:

- a non-therapeutic, and in particular cosmetic, method of photoprotecting human keratinous material against solar UV radiation;
- a method of coloring and/or lightening human keratinous material;
- a method of modifying the spectral reflectance of human keratinous material;

each of said methods comprising applying a cosmetic composition comprising a dispersion of photonic particles with a mean size in the range 1 μm to 500 μm, especially in the range 1 μm to 300 μm, each of said photonic particles comprising a diffracting arrangement of hollow nanoparticles.

The present description also describes a photonic particle for use in a method of photoprotection of human keratinous material against solar UV radiation wherein the photonic particle has a mean size in the range 1 μm to 500 μm, especially in the range 1 μm to 300 μm, and comprises a diffracting arrangement of hollow nanoparticles.

The present description also discloses a composition for use in a method of photoprotection of human keratinous material against solar UV radiation wherein the composition comprises a dispersion of photonic particles with a mean size in the range 1μm to 500μm, especially in the range 1μm to 300μm, and comprises a diffracting arrangement of hollow nanoparticles. The present description also discloses a composition, in particular a cosmetic composition, comprising a dispersion of photonic particles each having a form factor of less than 2, said particles comprising a diffracting arrangement of voids or monodisperse nanoparticles in a thermo-, electro- or photocrosslinkable matrix.

The present description also discloses:

- a non-therapeutic, and in particular cosmetic, method of photoprotecting human keratinous material against solar UV radiation;
- a method of coloring and/or lightening human keratinous material;
- a method of modifying the spectral reflectance of human keratinous material;

each of said methods comprising applying to said keratinous material a cosmetic composition comprising a dispersion of photonic particles each having a form factor of less than 2, said particles comprising a diffracting arrangement of voids or monodisperse nanoparticles in a thermo-, electro- or photocrosslinkable matrix.

The present description also discloses a composition for use in a method of photoprotection of human keratinous material against solar UV radiation wherein said composition comprises a dispersion of photonic particles each having a form factor of less than 2, said particles comprising a diffracting arrangement of voids or monodisperse nanoparticles in a thermo-, electro- or photocrosslinkable matrix.

The present description also discloses:

- a method of photoprotecting an ink, a paint, or a coating, comprising incorporating at least one composition comprising a dispersion of photonic particles as defined above into said ink or paint or said coating;
- a method of photoprotecting a material manufactured from at least one synthetic or natural polymer, comprising treating said polymer with a composition comprising a dispersion of photonic particles as defined above or comprising integrating at least said composition into said material;
- a method of photoprotecting an organic or mineral glass, comprising treating said glass with at least one composition comprising a dispersion of photonic particles as defined above or comprising integrating at least said composition into said glass;
- a method of photoprotecting a material comprising at least natural fibers and/or artificial fibers and/or synthetic fibers such as textiles or papers, comprising treating said material with at least one composition comprising a dispersion of photonic particles as defined above or comprising integrating at least said composition into said material.

The polymeric materials of the invention are, in particular plastic materials that are capable of being molded or worked, in general hot and under pressure, in order to result in a semimanufactured product or an article.

The polymers used for said materials are preferably selected from three major categories:

(i) <u>thermoplastics such as, for example:</u>

- acrylonitrile-butadiene-styrene (ABS) copolymers;
- cellulose acetate (CA) polymers;
- expanded polystyrenes (EPS);
- polystyrenes (PS);
- polyamides (PA);
- polybutylene terephthalate (PBT);
- polyethylene terephthalate (PET);
- polycarbonates (PC);
- polyethylenes (PE);
- polypropylenes (PP);
- polymethyl methacrylate (PMMA);
- polyformaldehydes (POM);
- polyvinyl acetates (PVAC);
- polyvinyl chlorides (PVC);
- styrene-acrylonitrile (SAN) copolymers;

(ii) <u>thermosets, such as :</u>

- polyepoxides (EP);
- melamine-formaldehyde (MF) copolymers;
- phenol-formaldehyde (PF) copolymers;
- cured polyurethanes(PUR);
- urea-formaldehyde (UF) copolymers;
- unsaturated polyesters.

(iii) <u>technical plastics such as:</u>

- polytetrafluoroethylene (PTFE).

Examples of natural fibers that may be mentioned, for example, are:

- plant fibers such as cotton, linseed, hemp, jute, straw, or latex;
- animal fibers such as wool, silk, mohair, angora, cashmere or alpaca.

Artificial fibers are generally obtained by chemical treatment (dissolution then precipitation) of natural substances such as milk caseins for lanital, or cellulose from various plants (pine bark, bamboo, soya, birch) for viscose. These chemical treatments are intended to produce a product that can be spun (capable of passing through the small holes of a die). At the die outlet, the filaments obtained are either combined to form continuous filaments in the manner of a silk thread, or are cut into discontinuous fibers in the manner of wool.

Examples of artificial fibers that may, for example, be mentioned are:

- cellulose acetate;
- cellulose triacetate; and
- viscose.

Particular examples of textile fibers are:

- polylactic acid;
- acrylic;
- aramides;
- elasthane: ®Lycra;
- chlorofiber;
- modacrylic;
- polyamide;
- polybenzimidazole;
- polyester;
- polyethylene;
- polyphenolic; and
- polyurea: polyurethane.

Examples of glasses that are suitable for use in the invention that may be mentioned are conventional mineral glasses based on silicates, glasses used for optics, glasses used for ophthalmology, especially organic glasses such as polycarbonates, for example bisphenol A polycarbonate or allyl polycarbonate, those used in the automobile industry (windshields), etc.

**Photonic particles**

In the context of the invention, photonic particles are also known as opals.

The photonic particles may have a form factor of less than 2, especially less than 1.75. When the particle is oblong, the form factor denotes the ratio of its largest longitudinal dimension to its largest transverse dimension. The photonic particles may be spherical, then having a form factor of 1.

A form factor of less than 2 may have an advantage in terms of surface coverage compared with flat particles that may become superimposed.

The mean size of the photonic particles may be in the range 1 μm to 500 μm, for example in the range 1 μm to 300 μm. The term "mean size" denotes the statistical grain-size dimension at half the population, termed D(0.5).

The photonic particles of the invention may comprise solid or hollow nanoparticles aggregated without a matrix or dispersed in any type of matrix, for example dispersed in a thermo-, electro- or photocrosslinkable matrix.

The content by weight of photonic particles may be in the range 0.1% to 20%, preferably in the range 1% to 10%, relative to the total composition weight, before application.

The photonic particles of the invention may, according to the different embodiments, be categorized as direct, inverse or pseudo inverse opals, as described below.

The photonic particles may be colorless.

The photonic particles may be solid or hollow.

**Direct opals**

Photonic particles of the "direct opal" type employ an arrangement of solid, optionally composite nanoparticles.
The photonic particles may comprise nanoparticles aggregated without a matrix. Such a photonic particle 1 comprising nanoparticles 10 aggregated without a matrix is shown in Figure 1.
A first method of manufacturing such particles may, as described in the publication by S-H Kim et al, JACS, 2006, 128, 10897 - 10904, comprises a step of obtaining a water-in-oil type emulsion, the aqueous phase comprising monodisperse nanoparticles, followed by a step of obtaining photonic particles comprising a step of using microwaves to irradiate the emulsion that has been obtained.
As described in the publication by S-M Yang, Langmuir 2005, 21, 10416 - 10421, a second manufacturing step may comprise a step of aggregating $SiO_2$ or polystyrene nanoparticles under electrospray.
Photonic particles of the "direct opal" type may also be obtained by a method such as that described in the publication "Ordered macroporous titania photonic balls by micrometer-scale spherical assembly templating" by Li et al, J. Mater. Chem., 2005, 15, 2551 - 2556.
The photonic particles of the "direct opal" type may also comprise nanoparticles aggregated in a matrix 20, as illustrated in Figure 2, which are in contact with one another, or dispersed in a matrix 20 as shown in Figure 3.
Several methods in addition to those mentioned above may be suitable for manufacturing said photonic particles, especially the method of aggregating $SiO_2$ particles in a silicon matrix as described in Honeywell's application US 2003/0148088.
As described in the publication by D. Pine, Langmuir 2005, 21, 6669 - 6674, a second method may comprise a step of aggregation from an emulsion of PMMA nanoparticles.
The photonic particles of the "direct opal" type may comprise nanoparticles dispersed in a photo-, electro- or thermocrosslinkable matrix.
The advantage of using an organic photo-, electro- or thermocrosslinkable, especially photocrosslinkable or thermocrosslinkable, matrix is because it is possible to adjust the distance between the nanoparticles contained in the matrix in order to vary the optical properties of the photonic particle. This distance may be a function of the fraction by weight of the nanoparticles dispersed in the organic matrix, before photo-, electro- or thermocrosslinking, especially before photocrosslinking or thermocrosslinking. Said weight fraction is equal to the ratio of the weight of nanoparticles divided by the weight of matrix before thermocrosslinking, electro-crosslinking or photocrosslinking.
In a preferred implementation of the invention, this fraction of nanoparticles is in the range 1% to 90% by weight, preferably in the range 5% to 60% by weight.
This type of photonic particle may be obtained using several emulsification methods, for example those described in the publication by S-H Kim et al, Adv Mater 2008, 9999, 1 - 7, which employs silica particles dispersed in a photocrosslinkable ETPTA (ethoxylated trimethylolpropane triacrylate) resin that can be photopolymerized under UV, or in the publication "Ordered macroporous titania photonic balls by micrometer-scale spherical assembly templating" by Li et al, J. Mater. Chem., 2005, 15, 2551 - 2556.
In some embodiments, the photonic particles comprise nanoparticles of polystyrene (PS) aggregated in a silicon matrix.
In some embodiments, the photonic particles comprise nanoparticles of polystyrene (PS) dispersed in a thermo-, electro- or photo-crosslinkable silicone resin.

**Inverse opals**

Photonic particles of the "inverse opal" type comprise holes instead of nanoparticles.
They may be obtained from direct opals after destroying nanoparticles, for example by calcining or acid hydrolysis, for example with 5% hydrofluoric acid, therewith leaving empty spaces in the place of all or part of the nanoparticles. The destruction step may possibly cause a reduction in the size of the fingerprint of the nanoparticle in the matrix, by up to 50%.
Calcining (500°C or 1000°C) may be carried out on direct opals based on organic nanoparticles and an inorganic matrix.
Acid hydrolysis, for example with a hydrofluoric acid solution, may be carried out on opals based on inorganic nanoparticles and an organic matrix.
With inverse opals, the ratio of the volume occupied by the nanoparticles divided by the volume occupied by the matrix (organic or precursor of the inorganic matrix) may vary over the range 99/1 to 80/20, thereby having the effect of causing the surface porosity of the inverse opals to vary. Such a variation is presented in the publication by D. Pine, F. Lange, Langmuir 2005, 21, 6669 - 6674.
The inverse opals may be produced using the methods described above for direct opals comprising nanoparticles aggregated or dispersed in a matrix, followed by a step of destroying nanoparticles, for example by calcining or

acid hydrolysis, for example as described in the following publications:

- A. Stein: Chem. Mater. 2002,14, 3305 - 3315, wherein opals are produced from monodisperse particles in zirconium acetate matrixes for ZrO articles, from Ti propoxide for $TiO_2$ opals, or from tetramethoxysilane (TMOS) for silica opals. After calcining, the PS particles give way to voids. The final material is then milled to produce opal powder;
- D. Pine, F. F. Lange: Langmuir, Vol 21, 15, 2005,6669 - 6674, describing the production of opals in the form of spheres using a method of emulsification followed by a step of calcining PMMA particles. The porosity of the opal is controlled by the ratio: Ti alkoxides divided by the amount of PMMA particles;
- F. F. Lange, Colloid Polym Sci (2003) 282, 7 - 13, describing the emulsification of particles of PMMA in the presence of Ti butoxide then calcining the PMMA particles.

By their nature, inverse opals are, in the absence of additional treatment, porous materials having optical properties that vary as a function of the medium which can fill the holes in the opals.
In order to guarantee their optical properties in any medium, photonic particles with an inverse opal structure may be coated and sealed against the medium into which they are immersed.
Said coating may, for example, be carried out using polymers or waxes.
Several methods are possible:

- spray drying: the principle is to dissolve or disperse (for latexes) the material that is to coat the photonic particles in a volatile solvent with an evaporation point of 100°C or less (ethanol, acetone, isopropanol, water, etc, or mixtures thereof). Spraying the ensemble into a chamber heated to a temperature that allows evaporation of the solvent or the mixture results in the material being deposited to coat the particles. These are entrained in a stream of air in a container at ambient temperature, for collection therefrom. An example that may be mentioned is the publication "Effects of fabrication conditions on the characteristics of etamidazole spray dried microspheres": Wang et al, J. Microencapsulation, 2002, vol.19, No 4, 495 - 510;
- bed of fluidized air: the fluidized air bed method is a method that is frequently used to dry and manufacture granules. A temperate stream of air is introduced via the bottom of a reactor. The suspension, sprayed by an atomizer into the production chamber, makes the particles in suspension bigger and they fall to the bottom whereupon they cannot be lifted by the stream of air.

In a non-limiting manner, the materials for coating the particles may be selected from the following:

- waxes and fats with a melting point of more than 45°C, especially carnauba wax, beeswax, stearyl stearate, polyethylene wax, DI 18/22 adipate, pentaerythrityl tetrastearate, tetracontanyl stearate, or dioctadecyl carbonate;
- cellulose and cellulose derivatives, in particular ethylcellulose, hydroxypropyl cellulose, hydroxypropylmethyl cellulose, hydroxybutyl cellulose, or polymers sold under the trademark Ethocel®;
- polycaprolactone having a molecular weight in the range 10000 g/mol [gram/mole] to 80000 g/mol;
- polylactic acid (PLA) and polylactic acid-glycolic acid (PLAGA) at a ratio lying in the range 90/10 to 50/50;
- polyvinyl alcohol;
- copolymers of polyvinylpyrrolidone and vinyl acetate; and copolymers of acrylic acid and methyl methacrylate sold under the trademark Eudragit® L100.

The weight ratio between the core of the photonic particle and the shell produced therewith may be in the range 99.9/0.1 to 80/20, and preferably in the range 99/1 to 90/10.
In exemplary embodiments, the photonic particles comprising nanoparticles aggregated without a matrix may optionally be coated by a coating, for example as described above.

**Pseudo-inverse opals**

"Pseudo-inverse" type photonic particles comprise hollow nanoparticles, aggregated without a matrix or dispersed in any type of matrix, for example dispersed in a thermo-, electro- or photo-crosslinkable matrix.
Producing direct opals from hollow nanoparticles, also termed "pseudo-inverse opals", has the advantages of amplifying the optical effects by a higher index difference compared with direct opals not using hollow nanoparticles and of offering a zero porosity compared with uncoated inverse opals, of optical properties that are dependent on the medium in which they are dispersed.
The hollow nanoparticles may be as described below.

### Janus type photonic particles

The photonic particles may be of the Janus type, i.e. comprising at least one other diffracting arrangement of nanoparticles, or even at least two other diffracting arrangements, the arrangements each having their own optical properties, especially different diffraction spectra.
In first exemplary embodiments, one arrangement may comprise solid nanoparticles and another arrangement may comprise solid or hollow nanoparticles.
In a variation, one arrangement may comprise hollow nanoparticles and another arrangement may comprise hollow nanoparticles.
When the particles comprise a plurality of arrangements, each arrangement may, for example, cover a different part of the UV spectrum, in order to obtain broader photoprotection.
The photonic particles comprising a plurality of diffracting arrangements may be obtained as taught in the publication by S-H Kim et al, Adv. Mater. 2008, 9999, 1 - 7 or in the publication "Patterned colloidal photonic domes and balls derived from viscous photocurable suspensions" from Kim et al, Adv. Mater. 2008, 20, 3211 - 3217.
When the photonic particles are used at least in part for their coloring properties, in particular to make the complexion uniform, the arrangements of nanoparticles, when illuminated with white light, may produce different respective colors; the arrangements may in particular produce a red, green and/or blue color, thereby enabling a large number of shades to be obtained, in particular white by additive synthesis of reflected light.
An arrangement presents a reflected red color, for example, when the reflectance in the visible spectrum is at least 50% in the wavelength range 620 nm to 700 nm, for an angle of observation in the range 30° to 150°. For green, the wavelength range under consideration is 490 nm to 550 nm, and for blue, it is 410 nm to 490 nm. The arrangements may diffract light through the various respective zones of the photonic particle, for example two opposed zones, for example two diametrically opposed hemispherical zones for a photonic particle that is spherical.
One of the arrangements may have a diffraction spectrum having at least one first order reflection peak in the wavelength range 250 nm to 400 nm and another arrangement may have a diffraction spectrum having at least one first order reflection peak in the range 250 nm to 400 nm or in the range 400 nm to 700 nm.

### Mixture of photonic particles

The composition of the invention may comprise a single type of photonic particle or a mixture of at least two different types of photonic particles, for example having reflection peaks, especially first order peaks, centered on different wavelengths located in the visible or UV region.
The composition may, for example, comprise a mixture of one type of photonic particles comprising solid nanoparticles and another type of photonic particles comprising nanoparticles that may be solid or hollow.
The composition may, for example, comprise a mixture of one type of photonic particle comprising hollow nanoparticles and another type of photonic particle comprising nanoparticles that may be hollow.
The composition may, for example, comprise a mixture of one type of photonic particle comprising a thermo-, electro- or photo-crosslinkable matrix and another type of photonic particle not comprising a thermo-, electro-or photo-crosslinkable matrix.

### Nanoparticles

The photonic particle nanoparticles may have a mean size in the range 100 nm to 500 nm, preferably in the range 100 nm to 300 nm. The term "mean size" denotes the statistical granulometric dimension at half the population, termed D(0.5).
The nanoparticles may be spherical in shape.
The nanoparticles may be 15% monodisperse or better. The term "x% monodisperse" as used in the invention means particles with a mean size having a coefficient of variation, CV, of x% or less. The coefficient of variation CV is defined by the relationship $CV=\frac{s}{D}$, s being the standard deviation of the particle size distribution and D being the mean size thereof. The mean size D and the standard deviation s may be measured for 250 particles by analyzing an image obtained using a scanning electron microscope, for example that with reference S-4 500 from the supplier HITACHI. Image analysis software may be used to facilitate this measurement, for example Winroof® software, from the supplier Mitani Corporation. Preferably, the coefficient of variation of the monodisperse nanoparticles is 10% or less, more preferably 7% or less, or even more preferably 5% or less, for example substantially of the order of 3.5% or less.
The nanoparticles may be solid or hollow, organic or inorganic.
The nanoparticles may be monolithic or composite.

When the monodisperse nanoparticles are composites, they may, for example, comprise a core and a shell produced from different substances, for example organic and/or mineral substances.

*Inorganic nanoparticles*

The nanoparticles may comprise an inorganic compound, or even be entirely mineral.
When the nanoparticles are inorganic, they may, for example, comprise at least one oxide, especially a metallic oxide, for example selected from silica, oxides of silica, iron, titanium, aluminum, chromium, zinc, copper, zirconium and cerium, and mixtures thereof. The nanoparticles may also include a metal, especially titanium, silver, gold, aluminum, zinc, iron, copper and mixtures and alloys thereof.

*Organic nanoparticles*

The nanoparticles may include an organic compound, or even be entirely organic.
Materials that may be suitable for producing organic nanoparticles that may be mentioned are polymers, in particular with a carbon or silicone chain, for example polystyrene (PS), polymethyl methacrylate (PMMA), polyacrylamide (PAM), silicone polymers, NADs ("non aqueous dispersions") such as rigid NADs for example, e.g. constituted by 96.7% methyl methacrylate and 3.3% ethylene glycol dimethacrylate 20% cured in isododecane, particle diameter: 141 nm (polydispersity Q = 0.14); or 90% methyl methacrylate and 10% allyl methacrylate, particle diameter: 170 nm; or 100% methyl dimethacrylate, particle diameter: 138 nm (polydispersity Q = 0.15); or poly(methyl methacrylate/ allyl methacrylate), polylactic acid (PLA), the polylactic acid-glycolic acid (PLAGA), celluloses and derivatives thereof, polyurethane, polycaprolactone, latex form, chitin, or composite chitin materials.
The glass transition temperature (Tg) of the organic nanoparticles may be greater than 40°C, and preferably greater than 60°C.

*Hollow nanoparticles*

These nanoparticles comprise a core and a shell. The shell may be organic or inorganic.
The shell of the nanoparticles may, for example, be formed from PS and the particles may, for example, be aggregated in an organic matrix.
The shell of the nanoparticles may, for example, be formed from PS and the particles may, for example, be dispersed in a thermo-, electro- or photo-crosslinkable organic matrix.
The core of said hollow nanoparticles may be constituted by air or a gas other than air in order to benefit from a different refractive index, for example $CO_2$, $N_2$, butane, or isobutane.
The presence of air or another gas inside the hollow nanoparticles may be used to obtain a large difference in the refractive index between the nanoparticles and the surrounding medium, which is favorable in terms of the intensity of the diffraction peak.
When the nanoparticles are hollow, the difference in refractive index at a wavelength diffracted between the core and the shell may be 0.4 or more. Said diffracted wavelength may be in the range 250 nm to 800 nm, for example in the range 250 nm to 400 nm. When the nanoparticles are hollow, the ratio between a largest dimension of the core and a largest dimension of the nanoparticle may be in the range 0.5 to 0.8. When the nanoparticles are hollow, the volume of the core represents in the range 10% to 80%, preferably in the range 20% to 60% of the total volume of the nanoparticle.
The thickness of the shell of the hollow nanoparticles, equal to half the difference between the largest dimension of the nanoparticle and the largest dimension of the core of the nanoparticle, may be in the range 50 nm to 200 nm, for example in the range 30 nm to 100 nm.
An example of hollow nanoparticles that may be mentioned are 280 nm nanoparticles from the supplier JSR SX866(B).
The core of the nanoparticles may optionally comprise a sunscreen or a mixture of sunscreens.

**Matrix**

The photonic particles may comprise hollow or solid nanoparticles, aggregated or dispersed in any type of matrix, for example dispersed in a thermo-, electro- or photo-crosslinkable matrix, or voids dispersed in any type of matrix, for example dispersed in a thermo-, electro- or photo-crosslinkable matrix, as mentioned above.
The matrix may be organic or inorganic.
Non-limiting examples of organic matrixes that may be mentioned include acrylic matrixes: polymethyl methacrylate (PMMA) or polacrylamide (PAM), matrices of polyethylene terephtalate (PET), polystyrene (PS), polycaprolactone (PCL), polyvinyl acetate(PVA), polyvinylethyl acetate (PVEA), and waxes with a melting point greater than 65°C [celsius], for example greater than 75°C, and with a hardness of more than 5 MPa and preferably more than 6 MPa [megapascal].

In particular, the matrix may be thermo-, photo- or electro-crosslinkable.

The term "photocrosslinkable matrix" means a matrix in which crosslinking is induced and/or assisted by light, especially UV.

The term "thermocrosslinkable matrix" means a matrix in which crosslinking is induced and/or assisted by adding heat, for example bringing the matrix to a temperature of more than 60°C.

The term "electrocrosslinkable matrix" means a matrix in which crosslinking is induced and/or assisted by applying an electric field.

A matrix may be both thermocrosslinkable and photocrosslinkable.

The photonic particles may comprise solid or hollow nanoparticles, dispersed in a thermo-, electro- or photo-crosslinkable matrix, or voids dispersed in a thermo-, electro- or photo-crosslinkable matrix.

The thermocrosslinkable or photocrosslinkable matrix may be organic.

Non-limiting examples of crosslinkable organic matrixes that may be mentioned are:

- photocrosslinkable polymers such as ETPA (ethoxylated trimethylolpropanetriacrylate), PEGDA (polyethyleneglycol diacrylate), acrylic resins, PEG diacrylates, or materials described in FR 2 833 487;
- copolymers, described in FR 2 848 428, which crosslink by polycycloaddition, of PVA or PVEA and of styrylpyridiniums with the following formulae:

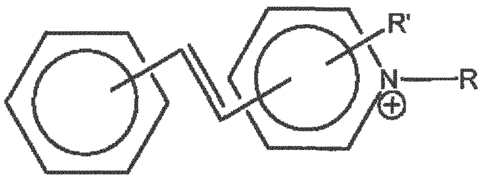

  where R represents a hydrogen atom, an alkyl group or a hydroxyalkyl group, and R' represents a hydrogen atom or an alkyl group;
- reactive silicones described in patent FR 2 910 286, i.e.:
  - polyorganosiloxanes comprising siloxane units with formula:

$$R_m R' SiO_{\frac{3-m}{2}}$$

    where R is a linear or cyclic monovalent hydrocarbon group containing 1 to 30 carbon atoms, m is equal to 1 or 2 and R' is an unsaturated aliphatic hydrocarbon group containing 2 to 10 carbon atoms or an unsaturated cyclic hydrocarbon group containing 5 to 8 carbon atoms; and/or
  - polyorganosiloxanes comprising at least one alkylhydrogenosiloxane unit with formula:

$$R_p HSiO_{\frac{3-p}{2}}$$

    where R is a linear or cyclic monovalent hydrocarbon group containing 1 to 30 carbon atoms or a phenyl group and p is equal to 1 to 2; and
- thermoplastic, thermocrosslinkable, electrocrosslinkable polymers.

The matrix crosslinking may be chemical crosslinking, for example using succinimides as described in application WO 2007/082061 A2.

For photocrosslinkable matrixes requiring a photoinitiator, the photoinitiator is selected, for example, from the following list: DMPA (dimethoxy 2-phenyl acetophenone), 2-benzyl-2-(dimethylamino)-1-[4-(4-morpholino phenyl]-l-butanone sold under the trademark Irgacure® 369 by Ciba®, 4,4'-bis(diethylamino)benzophenone sold by Sigma-Aldrich®, 2-hydroxy-4'-(2-hydroxyethoxy)-2-methylpropiophenone sold by Sigma-Aldrich®, 2-benzyl-2-(dimethylamino)-4'-morpholinobutyrophenone sold by Sigma-Aldrich®, phenylbis(2,4,6-trimethylbenzoyl)-phosphine oxide sold by Sigma-Aldrich®, isopropyl-thioxanthone sold by Sigma-Aldrich®, and camphorolactone.

The PEG diacrylates may, for example, be crosslinked using a photoinitiator such as camphorolactone.

Examples of inorganic matrixes that may be mentioned are metallic oxide matrixes, especially $SiO_2$, $TiO_2$, ZrO or $CaCO_3$, or Si matrixes.

## Medium containing photonic particles

The photonic particles may be contained in a cosmetically acceptable medium, i.e. a non-toxic medium suitable for application to human keratinous materials, in particular the skin, mucous membranes, or the hair or the nails.

Said medium is adapted to the nature of the support onto which the composition is to be applied and to the form in which the composition is to be packaged.

The medium may comprise a phase that is liquid at 25°C, containing photonic particles.

The medium may be selected so as to encourage dispersion of the photonic particles in the medium before application thereof, in order to prevent the photonic particles from becoming aggregated. As an example, it may be possible to use one or more agents that reduce the surface tension of the medium containing the photonic particles to less than 35 mN/m [millinewtons per meter].

The medium may be aqueous, with the photonic particles contained in an aqueous phase. The term "aqueous medium" denotes a medium that is liquid at ambient temperature and atmospheric pressure and contains a large fraction of water relative to the total weight of the medium. The complementary fraction may contain or be constituted by cosmetically acceptable organic solvents, miscible with water, for example alcohols or alkylene glycols. The quantity of water in the aqueous medium is, for example, 30% by weight or more, preferably 40% or more preferably 50%.

The medium may be monophase or multiphase.

The medium may comprise an alcohol, such as ethanol or isopropanol, for example, or a glycol derivative, in particular ethylene glycol or propylene glycol.

The medium may be transparent or translucent, and colored or not colored. The medium containing the photonic particles may contain no pigment or colorant. The coloration of the medium may be due to adding an additional coloring agent.

The medium may comprise a volatile solvant. The term "volatile solvant" as used in the context of the invention means any liquid capable of evaporating in contact with keratinous materials, at ambient temperature and at atmospheric pressure.

The medium may in particular be selected such that the composition contains at least 5%, or even at least 30% of volatile solvant.

The medium may comprise a film-forming polymer improving protection persistence.

### Film-forming polymer

In the present invention, the term "film-forming polymer" means a polymer that, by itself or in the presence of an auxiliary film-forming agent, is capable of forming a macroscopically continuous film that adheres to keratinous material, preferably a cohesive film, and more preferably a film of cohesion and mechanical properties that are such that said film can be isolated and manipulated in isolation, for example when said film is produced by casting over a non-stick surface such as a Teflon or silicone surface.

The composition may comprise an aqueous phase and the film-forming polymer may be present in said aqueous phase. It is then preferably a polymer in dispersion or an amphiphilic or associative polymer.

The term "polymer in dispersion" means polymers that are insoluble in water, in the form of particles of various sizes. The polymer may optionally be cured. The mean particle size is typically in the range 25 nm to 500 nm, preferably in the range 50 nm to 200 nm. The following polymers in aqueous dispersion may be used: Ultrasol 2075® from Ganz Chemical, Daitosol 5000AD® from Daito Kasei, Avalure UR 450® from Noveon, DYNAMX® from National Starch, Syntran 5760® from Interpolymer, Acusol OP 301® from Rohm&Haas, and Neocryl A 1090® from Avecia.

Acrylic dispersions sold under the names Neocryl XK-90®, Neocryl A-1070®, Neocryl A-1090®, Neocryl BT-62®, Neocryl A-1079® and Neocryl A-523® by the supplier AVECIA-NEORESINS, Dow Latex 432® by the supplier DOW CHEMICAL, Daitosol 5000 AD® or Daitosol 5000 SJ® by the supplier DAITO KASEY KOGYO, Syntran 5760® by the supplier Interpolymer, Allianz OPT by the supplier ROHM & HAAS, aqueous dispersions of acrylic or styrene/acrylic polymers sold under the trade name JONCRYL® by the supplier JOHNSON POLYMER, or aqueous dispersions of polyurethane sold under the names Neorez R-981® and Neorez R-974® by the supplier AVECIA-NEORESINS, Avalure UR-405®, Avalure UR-410®, Avalure UR-425®, Avalure UR-450®, Sancure 875®, Sancure 861®, Sancure 878® and Sancure 2060® by the supplier GOODRICH, Impranil 85® by the supplier BAYER, Aquamere H-1511® by the supplier HYDROMER, sulfopolyesters sold under the trade name Eastman AQ® by the supplier Eastman Chemical Products,

vinyl dispersions such as Mexomere PAM® from the supplier CHIMEX and mixtures thereof, are other examples of aqueous dispersions of particles of hydrodispersible film-forming polymers.

The term "amphiphilic or associative polymers" means polymers comprising one or more hydrophilic portions that render them partially soluble in water and one or more hydrophobic portions via which the polymers associate or interact. The following associative polymers may be used: Nuvis FX1100® from Elementis, Aculyn 22®, Aculyn 44®, Aculyn 46® from Rohm&Haas, and Viscophobe DB1000® from Amerchol. Diblock copolymers constituted by a hydrophilic block (polyacrylate, polyethylene glycol) and a hydrophobic block (polystyrene, polysiloxane) may also be used.

The composition may comprise an oily phase and the film-forming polymer may be present in said oily phase. The polymer may then be in dispersion or in solution. NAD type polymers or microgels (for example KSGs) may be used, as well as polymers of the PS-PA type or copolymers based on styrene (Kraton, Regalite).

Examples of non-aqueous dispersions of polymer particles in one or more silicone and/or hydrocarbon oils that can be stabilized at their surface by at least one stabilizing agent, in particular a block, graft or random polymer and that may be mentioned are acrylic dispersions in isododecane, such as Mexomere PAP® from the supplier CHIMEX, and dispersions of particles of a graft ethylenic polymer, preferably acrylic, in a liquid fatty phase, the ethylenic polymer advantageously being dispersed in the absence of additional stabilizer on the surface of particles such as that described in particular in the document WO 04/055081.

Film-forming polymers that may be used in the composition of the present invention and that may be mentioned are synthetic polymers of the radical or polycondensation type, polymers of natural origin, and mixtures thereof.

In particular, the radical type film-forming polymers may be polymers or copolymers, which are vinyls, especially acrylic polymers.

Vinyl film-forming polymers may result from polymerizing monomers containing an ethylenically unsaturated bond having at least one acid group and/or esters of said acid monomers and/or amides of said acid monomers, such as unsaturated $\alpha,\beta$-ethylenically unsaturated carboxylic acids, e.g. acrylic acid, methacrylic acid, crotonic acid, maleic acid or itaconic acid.

The polymers of natural origin, optionally modified, may be selected from shellac resin, sandarac gum, dammars, elemis, copals, and cellulose polymers such as nitrocellulose, ethylcellulose or nitrocellulose esters selected, for example, from cellulose acetate, cellulose acetobutyrate, cellulose acetopropionate, and mixtures thereof.

The film-forming polymer may be present in the form of solid particles in aqueous or oily dispersion, generally known as a latex or pseudolatex. The film-forming polymer may comprise one or more stable dispersions of particles of generally spherical polymers of one or more polymers, in a physiologically acceptable liquid fatty phase. These dispersions are generally termed polymer NADs, as opposed to latexes that are aqueous polymer dispersions. These dispersions may be in the form of nanoparticles of polymers in stable dispersion in said fatty phase. The nanoparticle size is preferably in the range 5 nm to 600 nm. The techniques for preparing said dispersions are well known to the skilled person.

The composition may comprise at least one film-forming polymer that is a linear, block, ethylenic film-forming polymer. Said polymer may comprise at least one first sequence (block) and at least one second sequence having different glass transition temperatures (Tg), said first and second sequences being connected together via an intermediate sequence comprising at least one constitutive monomer of the first sequence and at least one constitutive monomer of the second sequence. As an example, the first and second sequences and the block polymer may be incompatible with each other. Such polymers, for example, are described in the documents EP 1 411 069 or WO 04/028488, which are herewith incorporated by reference.

Fatty Phase

Although the composition containing the photonic particles may be free of oil, in some implementations the composition of the invention may include a fatty phase. The photonic particles may optionally be contained in said fatty phase.

The fatty phase may in particular be volatile.

The composition may comprise an oil such as, for example, synthesized esters or ethers, linear or branched hydrocarbons of mineral or synthetic origin, fatty alcohols containing 8 to 26 carbon atoms, partially fluorinated hydrocarbon and/or silicone oils, silicone oils such as polymethylsiloxanes (PDMS), which may optionally be volatile, with a linear or cyclic silicone chain, which may be liquid or pasty at ambient temperature, and mixtures thereof; other examples are given below.

A composition in accordance with the invention may thus comprise at least one volatile oil.

*Volatile oils*

In the context of the present invention, the term "volatile oil" means an oil (or non-aqueous medium) that is capable of evaporating on contact with skin in less than one hour, at ambient temperature and at atmospheric pressure.

The volatile oil is a volatile cosmetic oil, liquid at ambient temperature, in particular having a non-zero vapor pressure, at ambient temperature and atmospheric pressure, in particular having a vapor pressure in the range 0.13 Pa to 40000 Pa ($10^{-3}$ mmHg to 300 mmHg), in particular in the range 1.3 Pa to 13000 Pa (0.01 mmHg to 100 mmHg), and more particularly in the range 1.3 Pa to 1300 Pa (0.01 mmHg to 10 mmHg).
The volatile hydrocarbon oils may be selected from hydrocarbon oils of animal or vegetable origin containing 8 to 16 carbon atoms, and in particular branched $C_8$-$C_{16}$ alkanes (also termed isoparaffins), such as isododecane (also termed 2,2,4,4,6-pentamethylheptane), isodecane, isohexadecane, and, for example, oils sold under the trade names Isopars® or Permethyls®.
Examples of volatile oils that may also be used are volatile silicones, for example linear or cyclic volatile silicone oils, especially those with a viscosity of ≤8 centistokes ($8 \times 10^{-6}$ $m^2/s$), especially containing 2 to 10 silicon atoms, in particular 2 to 7 silicon atoms, said silicones optionally comprising alkyl or alkoxy groups containing 1 to 10 carbon atoms. Examples of volatile silicone oils that may be used in the invention that may be mentioned are dimethicones with a viscosity of 5 cSt to 6 cSt, octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.
It is also possible to use fluorinated volatile oils such as nonafluoromethoxybutane or perfluoromethylcyclopentane, and mixtures thereof.
It is also possible to use a mixture of the oils mentioned above.

*Non-volatile oils*

A composition of the invention may comprise a non-volatile oil.
Within the context of the present invention, the term "non-volatile oil" means an oil having a vapor pressure of less than 0.13 Pa, in particular high molecular mass oils.
The non-volatile oils may in particular be selected from hydrocarbon oils, fluorinated where appropriate, and/or non-volatile silicone oils.
Examples of non-volatile hydrocarbon oils that may be suitable for implementing the invention that may in particular be mentioned are:

- hydrocarbon oils of animal origin;
- hydrocarbon oils of vegetable origin, such as phytostearyl esters, for example phytostearyl oleate, physostearyl isostearate or lauroyl / octyldodecyl / phytostearyl glutamate sold, for example, under the name ELDEW PS203 by AJINOMOTO, triglycerides constituted by esters of fatty acids and glycerol in which the fatty acids may have chain lengths in the range $C_4$ to $C_{24}$, and may be linear or branched, saturated or unsaturated; said oils are in particular heptanoic or octanoic triglycerides, or wheatgerm, sunflower, grapeseed, sesame, corn, apricot, castor, shea, avocado, olive, soya, sweet almond, palm, rape, cottonseed, hazelnut, macadamia nut, jojoba, alfalfa, poppy, Hokaido squash, gourd, blackcurrant, evening primrose, millet, barley, quinoa, rye, carthame, bancoulier, passiflora, or musk rose oils; shea butter; or caprylic / capric acid triglycerides such as those sold by the supplier STEARINERIES DUBOIS OR those sold under the names MIGLYOL 810®, 812® and 818® by the supplier DYNAMIT NOBEL;
- hydrocarbon oils of mineral or synthetic origin such as, for example:

  o synthesized ethers containing 10 to 40 carbon atoms;
  o linear or branched hydrocarbons of mineral or synthetic origin, such as vaseline, polydecenes, hydrogenated polyisobutene such as parleam, squalane and mixtures thereof, and in particular hydrogenated polyisobutene; and
  o synthesized esters such as oils with formula $R_1COOR_2$, wherein $R_1$ represents the residue of a linear or branched fatty acid containing 1 to 40 carbon atoms and $R_2$ represents a hydrocarbon chain, especially branched, containing 1 to 40 carbon atoms, provided that $R_1 + R_2 \geq 10$.

The esters may be in particular be selected from esters, in particular of fatty acids such as, for example:

- cetostearyl octanoate, esters of isopropyl alcohol such as isopropyl myristate, isopropyl palmitate, ethyl palmitate, 2-ethyl-hexyl palmitate, isopropyl stearate or isostearate, isostearyl isostearate, octyl stearate, hydroxyl esters such as isostearyl lactate, octyl hydroxystearate, diisopropyl adipate, heptanoates, in particular isostearyl heptanoate, octanoates, decanoates or ricinoleates of alcohols or polyalcohols, such as propylene glycol dioctanoate, cetyl octanoate, tridecyl octanoate, 2-ethylhexyl 4-diheptanoate and palmitate, alkyl benzoate, polyethylene glycol diheptanoate, propyleneglycol 2-diethyl hexanoate and mixtures thereof, $C_{12}$-$C_{15}$ alcohol benzoates, hexyl laurate, neopentanoic acid esters such as isodecyl neopentanoate, isotridecyl neopentanoate, isostearyl neopentanoate, octyldodecyl neopentanoate, isononanoic acid esters such as isononyl isononanoate, isotridecyl isononanoate, octyl isononanoate, or hydroxyl esters such as isostearyl lactate, or di-isostearyl malate;

- esters of polyols, and pentaetrythritol esters, such as dipentaerythritol tetrahydroxystearat / tetraisostearate;
- esters of diol dimers and diacid dimers, such as Lusplan DD-DA5® and Lusplan DD-DA7®, sold by the supplier NIPPON FINE CHEMICAL and described in application FR 03 02809;
- fatty alcohols that are liquid at ambient temperature having a branched and/or unsaturated carbon chain containing 12 to 26 carbon atoms, such as 2-octyldodecanol, isostearyl alcohol, oleic alcohol, 2-hexyldecanol, 2-butyloctanol, or 2-undecylpentadecanol;
- higher fatty acids such as oleic acid, linoleic acid, linolenic acid and mixtures thereof; and
- dialkyl carbonates, the 2 alkyl chains possibly being identical or different, such as dicaprylyl carbonate sold under the name Cetiol CC®, by Cognis;
- non-volatile silicone oils such as, for example, non-volatile polydimethylsiloxanes (PDMS), polydimethylsiloxanes comprising pendant alkyl or alkoxy groups and/or with silicone chain ends, groups each containing 2 to 24 carbon atoms, phenyl silicones such as phenyl trimethicones, phenyl dimethicones, phenyl trimethylsiloxy diphenylsiloxanes, diphenyl dimethicones, diphenyl methyldiphenyl trisiloxanes, or 2-phenylethyl trimethylsiloxysilicates, or dimethicones or phenyltrimethicone with a viscosity less than or equal to 100 cSt, and mixtures thereof;
- and mixtures thereof.

The composition containing photonic particles may be free of oil, and in particular may contain no non-volatile oil.

**Complementary screens and additives**

The composition comprising photonic particles may comprise at least one additive selected from adjuvants that are normal in the cosmetics field, such as fillers, coloring agents, hydrophilic or lipophilic gelling agents, active ingredients, either hydrosoluble or liposoluble, preservatives, moisturizers such as polyols and in particular glycerin, sequestrating agents, antioxidants, solvents, fragrances, physical and chemical sunscreens, especially against UVA and/or UVB, odor absorbers, pH adjusting agents (acids or bases), and mixtures thereof.

The composition may contain at least one active ingredient having a complementary activity in the solar protection field, such as antioxidants, whitening agents in the context of anti-pigmentation and depigmentation, or anti-ageing active ingredients.

The additional organic screens, either hydrophobic, hydrophilic or insoluble in the usual solvents, may be selected from various categories of chemical compounds. In particular, the organic screens are selected from dibenzoylmethane derivatives; anthranilates; cinnamic derivatives; salicylic derivatives, camphor derivatives; benzophenone derivatives; β,β-diphenylacrylate derivatives; triazine derivatives other than those with formula (I); benzalmalonate derivatives, in particular those mentioned in patent US 5 624 663; benzimidazole derivatives; imidazolines; p-aminobenzoic acid (PABA) derivatives; benzotriazole derivatives; methylene bis-(hydroxyphenyl benzotriazole) derivatives such as those described in applications US 5 237 071, US 5 166 355, GB 2 303 549, DE 197 26 184 and EP 0 893 119; benzoxazole derivatives such as those described in patent applications EP 0 832 642, EP 1 027 883, EP 1 300 137 and DE 01 62 844; polymer screens and silicone screens such as those described in particular in application WO 93/04665; dimers derived from α-alkylstyrene, such as those described in patent application DE 198 55 649; 4,4-diarylbutadienes such as those described in applications EP 0 967 200, DE 197 46 654, DE 197 55 649, EP A 1 008 586, EP 1 133 980 and EP 0 133 981; or merocyanin derivatives such as those described in applications WO 04/006878, WO 05/058269 and WO 06/032741, and mixtures thereof.

The screen or screens may be selected from the following screens:

<u>Hydrophobic screens capable of absorbing UV in the range 320 nm to 400 nm (UVA)</u>

*<u>Dibenzoylmethane derivatives</u>*

Butyl methoxydibenzoylmethane sold in particular under the trade name "PARASOL 1789" by DSM Nutritional Products, Inc;

- Isopropyl dibenzoylmethane.

*<u>Aminobenzophenones</u>*

n-hexyl 2-(4-diethylamino-2-hydroxybenzoyl)-benzoate sold under the trade name "UVINUL A+" by BASF.

*Anthranilic derivatives*

Menthyl anthranilate sold under the trade name "NEO HELIOPAN MA" by SYMRISE.

*4,4-diarylbutadiene derivatives*

1,1-dicarboxy (2,2'-dimethyl-propyl)-4,4-diphenylbutadiene.

Preferred screens are butyl methoxydibenzoylmethane and n-hexyl 2-(4-diethylamino-2-hydroxybenzoyl)-benzoate.

Hydrophobic screens capable of adsorbing UV in the range 280 nm to 320 nm (UVB)

*Para-aminobenzoates*

Ethyl PABA;
Ethyl dihydroxypropyl PABA;
Ethylhexyl dimethyl PABA (ESCALOL 507 from ISP).

*Salicylic derivatives*

Homosalate sold under the name "Eusolex HMS" by Rona/EM Industries;

- Ethylhexyl salicylate sold under the name "NEO HELIOPAN OS" by SYMRISE;
- Dipropyleneglycol salicylate sold under the name "DIPSAL" by SCHER;
- TEA salicylate, sold under the name "NEO HELIOPAN TS" by SYMRISE.

*Cinnamates*

Ethylhexyl methoxycinnamate sold in particular under the trade name "PARSOL MCX" by DSM Nutritional Products, Inc;

- Isopropyl methoxy cinnamate;
- Isoamyl methoxy cinnamate sold under the trade name "NEO HELIOPAN E 1000" by SYMRISE;
- Diisopropyl methylcinnamate;
- Cinoxate;
- Glyceryl ethylhexanoate dimethoxycinnamate.

*β,β'-diphenylacrylate derivatives*

Octocrylene, sold in particular under the trade name "UVINUL N539" by BASF;

- Etocrylene, sold in particular under the trade name "UVINUL N35" by BASF.

*Benzylidene camphor derivatives*

3-Benzylidene camphor manufactured under the trade name "MEXORYL SD" by CHIMEX;

- Methylbenzylidene camphor sold under the trade name "EUSOLEX 6300" by MERCK;
- Polyacrylamidomethyl benzylidene camphor manufactured under the name "MEXORYL SW" by CHIMEX.

*Triazine derivatives*

Ethylhexyl triazone sold in particular under the trade name "UVINUL T150" by BASF;

- Diethylhexyl butamido triazone sold under the trade name "UVASORB HEB" by SIGMA 3V;
- 2,4,6-tris(dineopentyl 4'-amino benzalmalonate)-s-triazine;
- 2,4,6-tris-(diisobutyl 4'-amino benzalmalonate)-s-triazine;
- 2,4-bis(dineopentyl 4'-amino benzalmalonate)-6-(4'- n-butyl aminobenzoate)-s-triazine;

- 2,4-bis(n-butyl 4'-amino benzoate)-6-(aminopropyltrisiloxane)-s-triazine;
- symmetrical triazine screens described in patent US 6 225 467, application WO 2004/085412 (see compounds 6 to 9) or the document "Symmetrical Triazine Derivatives", IP.COM Journal, IP.COM INC WEST HENRIETTA, NY, US (20 September 2004), in particular 2,4,6-tris-(biphenyl)-1,3,5-triazine (in particular 2,4,6-tris(biphenyl-4-yl-1,3,5-triazine) and 2,4,6-tris(terphenyl)-1,3,5-triazine that is discussed in applications by BEIERSDORF, numbers WO 06/035000,

WO 06/034982, WO 06/034991, WO 06/035007, WO 2006/034992, WO 2006/034985.

*Imidazoline derivatives*

Ethylhexyl dimethoxybenzylidene dioxoimidazoline propionate.

*Benzalmalonate derivatives*

Polyorganosiloxanes having a benzalmalonate function, such as Polysilicone-15 sold under the trade name "PARSOL SLX" by DSM Nutritional Products, Inc;

- di-neopentyl 4'-methoxybenzalmalonate.

*Merocyanin derivatives*

Octyl-5-N,N-diethylamino-2-phenysulfonyl-2,4-pentadienoate.
Preferred screens are homosalate, ethylhexylsalicylate, octocrylene, ethylhexyl, methoxycinnamate v, isoamyl methoxycinnamate, ethylhexyl triazone, and diethylhexyl butamido triazone.
The most preferred compounds are ethylhexylsalicylate, octocrylene, ethylhexyl triazone and ethylhexyl methoxycinnamate.

Mixed hydrophobic screens capable of absorbing both UVA and UVB

*Benzophenone derivatives*

Benzophenone-1 sold under the trade name "UVINUL 400" by BASF;

- benzophenone-2 sold under the trade name "UVINUL D50" by BASF;
- Benzophenone-3 or oxybenzone, sold under the trade name "UVINUL M40" by BASF;
- benzophenone-5;
- benzophenone-6 sold under the trade name "Helisorb 11" by Norquay benzophenone-8 sold under the trade name "Spectra-Sorb UV-24" by American Cyanamid;
- benzophenone-10;
- benzophenone-11;
- benzophenone-12.

*Phenyl benzotriazole derivatives*

Drometrizole trisiloxane sold under the name "Silatrizole" by RHODIA CHIMIE;

- methylene bis-benzotriazolyl tetramethylbutylphenol, sold in the solid form under the trade name "MIXXIM BB/100" by FAIRMOUNT CHEMICAL or in micronized form in aqueous dispersion under the trade name "TINOSORB M" by CIBA SPECIALTY CHEMICALS.

*Bis-resorcinyl triazine derivatives*

Bis-ethylhexyloxyphenol methoxyphenyl triazine sold under the trade name "TINOSORB S" by CIBA GEIGY.

*Benzoxazole derivatives*

2,4-bis-[5-1(dimethylpropyl)benzoxazol-2-yl-(4-phenyl)-imino]-6-(2-ethylhexyl)-imino-1,3,5-triazine sold under

the name Uvasorb K2A by Sigma 3V.

The preferred screens are:

- drometrizole trisiloxane;
- methylene bis-benzotriazolyl tetramethylbutylphenol;
- bis-ethylhexyloxyphenol methoxyphenyl triazine; and
- benzophenone-3 or oxybenzone.

The most preferred are:

- drometrizole trisiloxane; and
- bis-ethylhexyloxyphenol methoxyphenyl triazine

Hydrosoluble screens capable of adsorbing UV in the range 320 nm to 400 nm (UVA)

Terephthalylidene dicamphor acid sulfonic acid manufactured under the name "MEXORYL SX" by CHIMEX.

Bis-benzoazolyl derivatives as described in patents EP 0 669 323 and US 2 463 264, more particularly the compound disodium phenyl dibenzimidazole tetrasulfonate sold under the trade name "NEO HELIOPAN AP" by Haarmann and REIMER.

The preferred screen is terephthalylidene dicamphor acid sulfonic acid.

Hydrosoluble screens capable of absorbing UV in the range 280 nm to 320 nm (UVB)

*p-aminobenzoic derivatives (PABA)*

PABA;

- glyceryl PABA; and
- PEG-25 PABA sold under the name "UVINUL P25" by BASF;
- phenylbenzimidazole sulfonic acid sold in particular under the trade name "EUSOLEX 232" by MERCK;
- ferulic acid;
- salicylic acid;
- DEA methoxycinnamate;
- benzylidene camphor sulfonic acid manufactured under the name "MEXORYL SL" by CHIMEX;
- camphor benzalkonium methosulfate manufactured under the name "MEXORYL SO" by CHIMEX; and
- The preferred screen is phenylbenzimidazole sulfonic acid.

Mixed UVA and UVB hydrosoluble screens

*Benzophenone derivatives comprising at least one sulfonic radical*

Benzophenone-4 sold under the trade name "UVINUL MS40" by BASF;

- benzophenone-5; and
- benzophenone-9.

The preferred screen is benzophenone-4.

The organic screen or screens of the invention may be present in the compositions of the invention in a concentration in the range 0.1% to 15%, preferably in the range 0.2% to 10%, by weight relative to the total composition weight.

Inorganic sunscreens or photoprotectors

The inorganic photoprotective agents are selected from metallic oxide pigments that may optionally be coated (mean size of primary particles: generally in the range 5 nm to 100 nm, preferably in the range 10 nm to 50 nm), such as titanium oxide pigments (amorphous or crystalline in the form of rutile and/or anatase), iron, zinc, zirconium, or cerium, which are all UV photoprotectors that are well known per se.

The pigments may optionally be coated.

The coated pigments are pigments that have undergone one or more surface treatments of a chemical, electronic, or chemical-mechanical nature with compounds such as those described in Cosmetics & Toiletries, February 1990, Vol 105, pp. 53 - 64, such as amino acids, beeswax, fatty acids, fatty alcohols, anionic surfactants, lecithins, sodium, potassium, zinc, iron, or aluminum salts of fatty acids, metallic (titanium or aluminum) alkoxides, polyethylene, silicones, proteins (collagen, elastin), alkanolamines, oxides of silicon, metallic oxides or sodium hexametaphosphate.

In known manner, the silicones are organo-silicon polymers or oligomers with a linear or cyclic, branched or cured structure, with a variable molecular weight obtained by polymerization and/or polycondensation of suitably functionalized silanes and essentially constituted by repeated principal motifs in which the silicon atoms are connected together via oxygen atoms (siloxane linkage), with hydrocarbon radicals that may be substituted being directly bonded via a carbon atom to said silicon atoms.

The term "silicones" also encompasses pigments that are necessary for their preparation, in particular alkylsilanes.

The silicones that are used for coating pigments for suitable use in the present invention are preferably selected from the group containing alkyl silanes, polydialkylsiloxanes and polyalkylhydrogenosiloxanes. Still more preferably, the silicones are selected from the group containing octyl trimethyl silane, polydimethylsiloxanes and polymethylhydrogenosiloxanes.

Clearly, prior to treating them with silicones, the metallic oxide pigments may have been treated with other surface agents, in particular with cerium oxide, alumina, silica, aluminum compounds, silicon compounds, or mixtures thereof.

More particularly, the coated pigments are titanium oxides coated with the following:

- silica, such as the product "SUNVEIL" from the supplier IKEDA;
- silica and iron oxide, such as the product "SUNVEIL F" from the supplier IKEDA;
- silica and alumina, such as the products "MICROTITANIUM DIOXIDE MT 500 SA" and "MICROTITANIUM DIOXIDE MT 100 SA" from the supplier TAYCA, or "TIOVEIL" from the supplier TIOXIDE;
- alumina, such as the products "TIPAQUE TTO-55 (B)" or "TIPAQUE TTO-55 (A)" from the supplier ISHIHARA, and "UVT 14/4" from the supplier KEMIRA;
- alumina and aluminum stearate, such as the products "MICROTITANIUM DIOXIDE MT 100 T, MT 100 TX, MT 100 Z, MT-01" from the supplier TAYCA, the product "Solaveil CT-10 W" and "Solaveil CT 100" from the supplier UNIQEMA and the product "Eusolex T-AVO" from the supplier MERCK;
- silica, alumina and alginic acid, such as the product "MT-100 AQ" from the supplier TAYCA;
- alumina and aluminum laurate, such as the product "MICROTITANIUM DIOXIDE MT 100 S" from the supplier TAYCA;
- iron oxide and iron stearate, such as the product "MICROTITANIUM DIOXIDE MT 100 F" from the supplier TAYCA;
- zinc oxide and zinc stearate, such as the product "BR 351" from the supplier TAYCA;
- silica and alumina treated with a silicone, such as the product "MICROTITANIUM DIOXIDE MT 600 SAS", "MICROTITANIUM DIOXIDE MT 500 SAS", or "MICROTITANIUM DIOXIDE MT 100 SAS" from the supplier TAYCA;
- silica, alumina, and aluminum stearate treated with a silicone, such as the products "STT-30-DS" from the supplier TITAN KOGYO;
- silica treated with a silicone, such as the product "UV-TITAN X 195" from the supplier KEMIRA;
- alumina treated with a silicone, such as the products "TIPAQUE TTO-55 (S)" from the supplier ISHIHARA, or "UV TITAN M 262" from the supplier KEMIRA;
- triethanolamine, such as the product "STT-65-S" from the supplier TITAN KOGYO;
- stearic acid, such as the product "TIPAQUE TTO-55 (C)" from the supplier ISHIHARA;
- sodium hexametaphosphate, such as the product "MICROTITANIUM DIOXIDE MT 150 W" from the supplier TAYCA;
- $TiO_2$ treated with octyl trimethyl silane, sold under the trade name "T 805" by the supplier DEGUSSA SILICES;
- $TiO_2$ treated with a polydimethylsiloxane, sold under the trade name "70250 Cardre UF TiO2SI3" by the supplier CARDRE;
- $TiO_2$ anatase/rutile treated with a polydimethylhydrogenosiloxane sold under the trade name "MICRO TITANIUM DIOXIDE USP GRADE HYDROPHOBIC" by the supplier COLOR TECHNIQUES.

Uncoated titanium oxide pigments are, for example, sold by the supplier TAYCA under the trade names "MICROTITANIUM DIOXIDE MT 500 B" or "MICROTITANIUM DIOXIDE MT600 B", by the supplier DEGUSSA under the name "P 25", by the supplier WACKER under the name "Transparent titanium oxide PW", by the supplier MIYOSHI KASEI under the name "UFTR", by the supplier TOMEN under the name "ITS" and by the supplier TIOXIDE under the name "TIOVEIL AQ".

Examples of uncoated zinc oxide pigments are:

- those sold under the name "Z-cote" by the supplier Sunsmart;
- those sold under the name "Nanox" by the supplier Elementis;

- those sold under the name "Nanogard WCD 2025" by the supplier Nanophase Technologies.

Examples of coated zinc oxide pigments are:

- those sold under the name "Zinc oxide CS-5" by the supplier Toshibi (znO coated with polymethylhydrogenosiloxane);
- those sold under the name "Nanogard Zinc Oxide FN" by the supplier Nanophase Technologies (40% dispersion in Finsolv TN, $C_{12}$-$C_{15}$ alcohol benzoate);
- those sold under the name "DAITOPERSION ZN-30" and "DAITOPERSION Zn-50" by the supplier Daito (dispersions in cyclopolymethylsiloxane / oxyethylenated polydimethylsiloxane, containing 30% to 50% of nano-zinc oxides coated with silica and polymethylhydrogenosiloxane);
- those sold under the name "NFD Ultrafine ZnO" by the supplier Daikin (ZnO coated with perfluoroalkyl phosphate and copolymer based on perfluoroalkylethyl in dispersion in cyclopentasiloxane);
- those sold under the name "SPD-Z1" by the supplier Shin-Etsu (ZnO coated with silicone-grafted acrylic polymer, dispersed in cyclodimethylsiloxane);
- those sold under the name "Escalol Z100" by the supplier ISP (ZnO-treated alumina dispersed in an ethylhexyl methoxycinnamate / PVP-hexadecene copolymer / methicone mixture);
- those sold under the name "Fuji znO-SMS-10" by the supplier Fuji Pigment (ZnO coated with silica and polymethylsilsesquioxane);
- those sold under the name "Nanox Gel TN" by the supplier Elementis (ZnO dispersed in a concentration of 55% in $C_{12}$-$C_{15}$ alcohol benzoate with polycondensate of hydroxystearic acid).

Uncoated cerium oxide pigments are sold under the name "COLLOIDAL CERIUM OXIDE" by the supplier RHONE POULENC.

Uncoated iron oxide pigments are sold, for example, by the supplier ARNAUD under the names "NANOGARD WCD 2002 (FE 45B)", "NANOGARD IRON FE 45 BL AQ", "NANOGARD FE 45R AQ", "NANOGARD WCD 2006 (FE 45R)", or by the supplier MITSUBISHI under the name "TY-220".

Coated iron oxide pigments are, for example, sold by the supplier ARNAUD under the names "NANOGARD WCD 2008 (FE 45B FN)", "NANOGARD WCD 2009 (FE 45B 556)", "NANOGARD FE 45 BL 345", "NANOGARD FE 45 BL", or by the supplier BASF under the name "TRANSPARENT IRON OXIDE".

Mention may also be made of mixtures of metallic oxides, in particular of titanium dioxide and of cerium dioxide, including the mixture of equal weights of titanium dioxide and cerium dioxide coated with silica, sold by the supplier IKEDA under the name "SUNVEIL A", as well as a mixture of titanium dioxide and zinc dioxide coated with alumina, silica and silicone, such as the product "M 261" sold by the supplier KEMIRA, or coated with alumina, silica and glycerin, such as the product "M 211" sold by the supplier KEMIRA.

The inorganic screen or screens may be present in the compositions of the invention in a concentration in the range 0.1% to 15%, preferably in the range 0.2% to 10%, by weight relative to the total composition weight.

The additive or additives may be selected from those mentioned in the CTFA Cosmetic Ingredient Handbook, 10th Edition, Cosmetic and Fragrance Assn, Inc., Washington DC (2004), herewith incorporated by reference.

## Galenical forms

The photonic particles may be used in lotions, creams, milks, pommades, gels, films, patches, sticks, powder, pastes, for the skin, the lips, the hair or the nails.

## Modes of application

The composition comprising photonic particles may be applied by hand or using an applicator.

Application may also be carried out by spraying or projection using a piezoelectric device, for example, or by transferring a layer of composition that has been deposited on an intermediate support.

## Packaging

The composition may be packaged in any packaging device, especially formed from thermoplastic material, or on any support provided for that purpose.

The packaging device may be a bottle, a pump bottle, an aerosol bottle, a tube, a sachet or a pot.

## Example

Nanoparticles are constituted by 100% of 285 nm PMMA nanoparticles from the supplier SOKEN.
The photonic particles, obtained by spray drying, were approximately 30 μm in size.
Figure 4 represents an absorption spectrum of various compositions comprising photonic particles of the invention.
Unless otherwise specified, the expression "comprising a" should be construed as meaning "comprising at least one".

## Claims

1. A composition for use in a method of photoprotecting human keratinous material against solar UV radiation, the composition comprising a dispersion of photonic particles with a mean size in the range 1 μm to 500 μm, each comprising a diffracting arrangement of monodisperse nanoparticles or voids, the diffraction spectrum of said arrangement including a first order reflection peak in the wavelength range 250 nm to 400 nm,
said photonic particles being substantially spherical in shape.

2. A composition according to claim 1, wherein the photonic particles comprise nanoparticles aggregated without a matrix.

3. A composition according to claim 1, wherein the photonic particles comprise nanoparticles (10), aggregated or dispersed in a matrix (20).

4. A composition according to any preceding claim,
wherein the mean size of the nanoparticles (10) is in the range 100 nm to 500 nm, preferably in the range 100 nm to 350 nm.

5. A composition according to any preceding claim,
wherein the composition includes an additional sunscreen and/or an additional coloring agent.

6. A composition according to any preceding claim,
wherein at least one photonic particle (1) includes at least one other diffracting arrangement of nanoparticles (10), especially two other arrangements, the arrangements having different diffraction spectra.

7. A composition according to any one of claims 3 to 6, wherein the matrix (20) is:

   - an organic matrix selected from:

     - acrylic polymethyl methacrylate (PMMA) or polyacrylamide (PAM) matrixes, polyethylene terephthalate (PET), polystyrene (PS), polycaprolactone, polyvinyl acetate, or polyvinylethyl acetate matrixes, and waxes with a melting point of more than 65°C and with a hardness of more than 5 MPa; and
     - organic crosslinkable matrixes selected from:

       - photocrosslinkable polymers such as ETPA (ethoxylated trimethylolpropanetriacrylate), PEGDA (polyethyleneglycol diacrylate), acrylic resins, PEG diacrylates,
       - copolymers of polyvinyl acetate or polyvinylethyl acetate and of styrylpyridiniums with the following formulae:

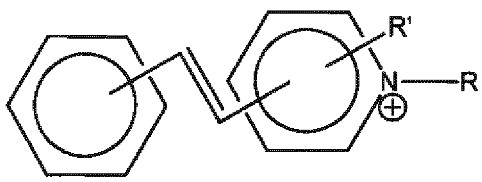

where R represents a hydrogen atom, or an alkyl or hydroxyalkyl group, and R' represents a hydrogen atom or an alkyl group;

- cinnamic acid derivatives such as polyvinylcinnamate or PDMS cinnamate;
- reactive silicones, i.e.:

- polyorganosiloxanes comprising siloxane units with formula:

$$R_m R' SiO_{\frac{3-m}{2}}$$

where R is a monovalent, linear or cyclic hydrocarbon group containing 1 to 30 carbon atoms, m is equal to 1 or 2, and R' is an unsaturated aliphatic hydrocarbon group containing 2 to 10 carbon atoms or an unsaturated cyclic hydrocarbon group containing 5 to 8 carbon atoms;
- polyorganosiloxanes comprising at least one alkylhydrogenosiloxane unit with formula:

$$R_p HSiO_{\frac{3-p}{2}}$$

where R is a monovalent, linear or cyclic, hydrocarbon group containing 1 to 30 carbon atoms or a phenyl group and p is equal to 1 to 2; and

- thermoplastic, thermocrosslinkable or electro-crosslinkable polymers;

• an inorganic matrix selected from metallic oxides, in particular $SiO_2$, $TiO_2$, ZrO, or an inorganic $CaCO_3$ or Si matrix.

**8.** A composition according to any one of claims 1 to 7, wherein the photonic particles (1) are colorless.

**9.** A composition according to any preceding claim, wherein the nanoparticles (10) have a mean size having a coefficient of variation lying in the range 5 % to 15 %.

**10.** A composition according to any preceding claim, wherein the photonic particles each have a mean size lying in the range 1 to 50 μm, better 1 to 40 μm, better 1 to 20 μm.

**11.** A composition according to any preceding claim, wherein the nanoparticles are inorganic and, in particular, comprise silica.

**12.** A composition according to any preceding claim, wherein the photonic particles are of the direct opal type.

**13.** A composition according to any one of claims 3 to 11, wherein the photonic particles are of the inverse opal type.

**14.** A composition according to any one of claims 3 to 13, wherein the matrix does not comprise additional nanoparticles, in particular of metal or metal oxide type, different from the nanoparticles constituting the diffracting arrangement.

## Patentansprüche

**1.** Zusammensetzung zur Verwendung in einem Lichtschutzverfahren von humanem keratinösem Material gegen UV-Sonnenstrahlung, wobei die Zusammensetzung eine Dispersion photonischer Teilchen mit einer mittleren Größe im Bereich von 1 μm bis 500 μm umfasst, jeweils umfassend eine Beugungsanordnung von monodispersen Nanopartikeln oder Hohlräumen, wobei das Beugungsspektrum der Anordnung einen Reflexionspeak erster Ordnung im Wellenlängenbereich von 250 nm bis 400 nm einschließt,
wobei die photonischen Teilchen im Wesentlichen eine kugelförmige Gestalt aufweisen.

**2.** Zusammensetzung gemäß Anspruch 1, wobei die photonischen Teilchen Nanopartikel, aggregiert ohne eine Matrix, umfassen.

**3.** Zusammensetzung gemäß Anspruch 1, wobei die photonischen Teilchen Nanopartikel (10), aggregiert oder dispergiert in einer Matrix (20), umfassen.

**4.** Zusammensetzung gemäß einem vorhergehenden Anspruch, wobei die mittlere Größe der Nanopartikel (10) im Bereich von 100 nm bis 500 nm, bevorzugt im Bereich von 100 nm bis 350 nm liegt.

**5.** Zusammensetzung gemäß einem vorhergehenden Anspruch, wobei die Zusammensetzung ein zusätzliches Sonnenschutzmittel und/oder ein zusätzliches farbgebendes Mittel einschließt.

**6.** Zusammensetzung gemäß einem vorhergehenden Anspruch, wobei mindestens ein photonisches Teilchen (1) mindestens eine weitere Beugungsanordnung von Nanopartikeln (10), insbesondere zwei weitere Anordnungen, wobei die Anordnungen unterschiedliche Beugungsspektren aufweisen, einschließt.

**7.** Zusammensetzung gemäß einem der Ansprüche 3 bis 6, wobei die Matrix (20) ist:

- eine organische Matrix, ausgewählt aus:

  - acrylischen Polymethylmethacrylat (PMMA)- oder Polyacrylamid (PAM)-Matrizes, Polyethylenterephthalat (PET)-, Polystyrol (PS)-, Polycaprolacton-, Polyvinylacetat- oder Polyvinylethylacetat-Matrizes, und Wachsen mit einem Schmelzpunkt von höher als 65°C und mit einer Härte von höher als 5 MPa; und
  - organischen vernetzbaren Matrizes, ausgewählt aus:

    - photovernetzbaren Polymeren wie ETPA (ethoxyliertes Trimethylolpropantriacrylat), PEGDA (Polyethylenglycoldiacrylat), acrylischen Harzen, PEG-Diacrylaten,
    - Copolymeren von Polyvinylacetat oder Polyvinylethylacetat und Styrylpyridiniumverbindungen mit den folgenden Formeln:

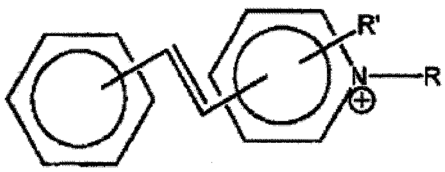

wobei R ein Wasserstoffatom oder einen Alkyl- oder Hydroxyalkylrest darstellt, und R' ein Wasserstoffatom oder einen Alkylrest darstellt;
    - Zimtsäurederivaten wie Polyvinylcinnamat oder PDMS-Cinnamat;
    - reaktiven Siliconen, d.h.:

      - Polyorganosiloxanen, umfassend Siloxaneinheiten mit der Formel:

$$R_m R' SiO_{\frac{3-m}{2}}$$

wobei R ein einwertiger, linearer oder cyclischer, 1 bis 30 Kohlenstoffatome enthaltender Kohlenwasserstoffrest ist, m gleich 1 oder 2 ist, und R' ein ungesättigter, aliphatischer, 2 bis 10 Kohlenstoffatome enthaltender Kohlenwasserstoffrest oder ein ungesättigter, cyclischer, 5 bis 8 Kohlenstoffatome enthaltender Kohlenwasserstoffrest ist;
      - Polyorganosiloxanen, umfassend mindestens eine Alkylhydrogenosiloxaneinheit mit der Formel:

$$R_p HSiO_{\frac{3-p}{2}}$$

wobei R ein einwertiger, linearer oder cyclischer, 1 bis 30 Kohlenstoffatome enthaltender Kohlenwasserstoffrest oder eine Phenylgruppe ist und p gleich 1 bis 2 ist; und
- thermoplastischen, thermovernetzbaren oder elektrovernetzbaren Polymeren;

• eine anorganische Matrix, ausgewählt aus metallischen Oxiden, insbesondere $SiO_2$, $TiO_2$, ZrO, oder einer anorganischen $CaCO_3$- oder Si-Matrix.

**8.** Zusammensetzung gemäß einem der Ansprüche 1 bis 7, wobei die photonischen Teilchen (1) farblos sind.

**9.** Zusammensetzung gemäß einem vorhergehenden Anspruch, wobei die Nanopartikel (10) eine mittlere Größe mit einem im Bereich von 5 % bis 15 % liegendem Variationskoeffizienten aufweisen.

**10.** Zusammensetzung gemäß einem vorhergehenden Anspruch, wobei die photonischen Teilchen jeweils eine mittlere Größe, die im Bereich von 1 bis 50 μm, besser 1 bis 40 μm, besser 1 bis 20 μm liegt, aufweisen.

**11.** Zusammensetzung gemäß einem vorhergehenden Anspruch, wobei die Nanopartikel anorganisch sind und insbesondere Siliciumdioxid umfassen.

**12.** Zusammensetzung gemäß einem vorhergehenden Anspruch, wobei die photonischen Teilchen vom Typ direkter Opal sind.

**13.** Zusammensetzung gemäß einem der Ansprüche 3 bis 11, wobei die photonischen Teilchen vom Typ inverser Opal sind.

**14.** Zusammensetzung gemäß einem der Ansprüche 3 bis 13, wobei die Matrix keine zusätzlichen Nanopartikel, insbesondere vom Typ Metall oder Metalloxid, welche verschieden von den Nanopartikeln, die die Beugungsanordnung bilden, sind, umfasst.

## Revendications

**1.** Composition pour utilisation dans un procédé de photoprotection des matières kératiniques humaines vis-à-vis du rayonnement UV solaire, la composition comportant une dispersion de particules photoniques de taille moyenne comprise entre 1 et 500 μm, comportant chacune un arrangement diffractant de nanoparticules monodispersées ou de vides, le spectre de diffraction de cet arrangement comportant un pic de réflexion du premier ordre dans la plage de longueurs d'ondes allant de 250 à 400 nm,
lesdites particules photoniques étant de forme sensiblement sphérique.

**2.** Composition selon la revendication 1, dans laquelle les particules photoniques comportent des nanoparticules agrégées sans matrice.

**3.** Composition selon la revendication 1, dans laquelle les particules photoniques comportent des nanoparticules (10) agrégées ou dispersées au sein d'une matrice (20).

**4.** Composition selon l'une quelconque des revendications précédentes, dans laquelle la taille moyenne des nanoparticules (10) est comprise entre 100 et 500 nm, mieux entre 100 et 350 nm.

**5.** Composition selon l'une quelconque des revendications précédentes, laquelle composition comporte un filtre solaire additionnel et/ou un agent de coloration additionnel.

**6.** Composition selon l'une quelconque des revendications précédentes, dans laquelle au moins une particule photonique (1) comporte au moins un autre arrangement diffractant de nanoparticules (10), notamment deux autres arrangements, les arrangements ayant des spectres de diffraction différents.

**7.** Composition selon l'une quelconque des revendications 3 à 6, la matrice (20) étant une :

• matrice organique choisie parmi :

- les matrices acryliques en polyméthacrylate de méthyle (PMMA) ou polyacrylamide (PAM), les matrices en polyéthylène téréphtalate (PET), en polystyrène (PS), en polycaprolactone, en polyvinyl acétate, en polyvinyléthyl acétate, les cires à point de fusion supérieur à 65°C et de dureté supérieure à 5 MPa, et
- les matrices organiques réticulables choisies parmi :

  - les polymères photoréticulables comme l'ETPA (Ethoxylated TrimethylolPropanetriAcrylate), le PEGDA (polyethyleneglycol diacrylate), les résines acryliques, les PEG diacrylates,
  - les copolymères de polyvinyl acétate ou de polyvinyléthyl acétate et de styrylpyridiniums de formules suivantes :

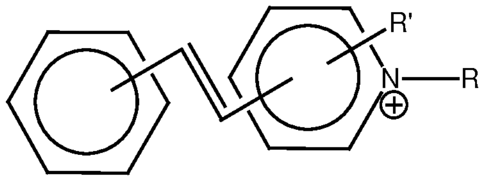

  où R représente l'atome d'hydrogène, un groupement alkyle ou hydroxyalkyle, et R' représente l'atome d'hydrogène ou un groupement alkyle ;
  - les dérivés d'acide cinnamique comme le polyvinylcinnamate et le PDMS cinnamate,
  - les silicones réactives, c'est-à-dire :

    - les polyorganosiloxanes comportant des unités siloxanes de formule : $R_m R' SiO_{\frac{3-m}{2}}$ dans laquelle R est un groupe hydrocarboné monovalent, linéaire ou cyclique, comportant de 1 à 30 atomes de carbone, m est égal à 1 ou 2 et R' est un groupement hydrocarboné aliphatique insaturé comportant de 2 à 10 atomes de carbone ou un groupement hydrocarboné cyclique insaturé comportant de 5 à 8 atomes de carbone ;
    - les polyorganosiloxanes comportant au moins une unité alkylhydrogénosiloxane de formule : $R_p HSiO_{\frac{3-p}{2}}$ où R est un groupe hydrocarboné monovalent, linéaire ou cyclique, comportant de 1 à 30 atomes de carbone ou un groupe phényle et p est égal à 1 ou 2 ; et
    - les polymères thermoplastiques, thermoréticulables, électroréticulables,

- matrice inorganique choisie parmi les oxydes métalliques, notamment $SiO_2$, $TiO_2$, ZrO ou une matrice inorganique en $CaCO_3$ ou en Si.

**8.** Composition selon l'une quelconque des revendications 1 à 7, les particules photoniques (1) étant incolores.

**9.** Composition selon l'une quelconque des revendications précédentes, dans laquelle les nanoparticules (10) ont une taille moyenne ayant un coefficient de variation situé dans la plage de 5 % à 15 %.

**10.** Composition selon l'une quelconque des revendications précédentes, dans laquelle les particules photoniques ont chacune une taille moyenne située dans la plage de 1 à 50 μm, de préférence de 1 à 40 μm, encore plus préférablement de 1 à 20 μm.

**11.** Composition selon l'une quelconque des revendications précédentes, dans laquelle les nanoparticules sont inorganiques et, en particulier, comprennent de la silice.

**12.** Composition selon l'une quelconque des revendications précédentes, dans laquelle les particules photoniques sont du type opale directe.

**13.** Composition selon l'une quelconque des revendications 3 à 11, dans laquelle les particules photoniques sont du type opale inverse.

**14.** Composition selon l'une quelconque des revendications 3 à 13, dans laquelle la matrice ne comprend pas de nanoparticules additionnelles, en particulier de type métal ou oxyde de métal, différentes des nanoparticules cons-

tituant l'arrangement diffractant.

Fig.1

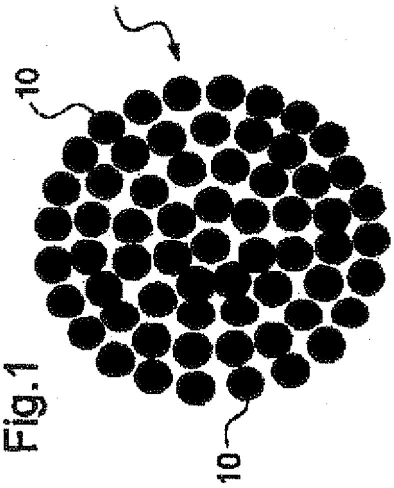


Fig.2

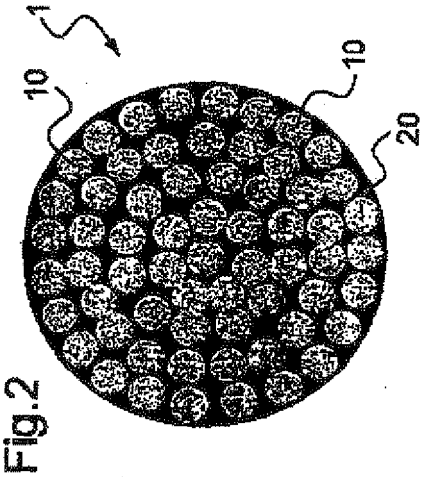


Fig.3

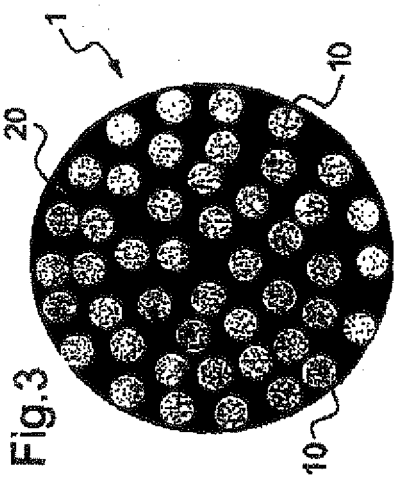


Fig.4

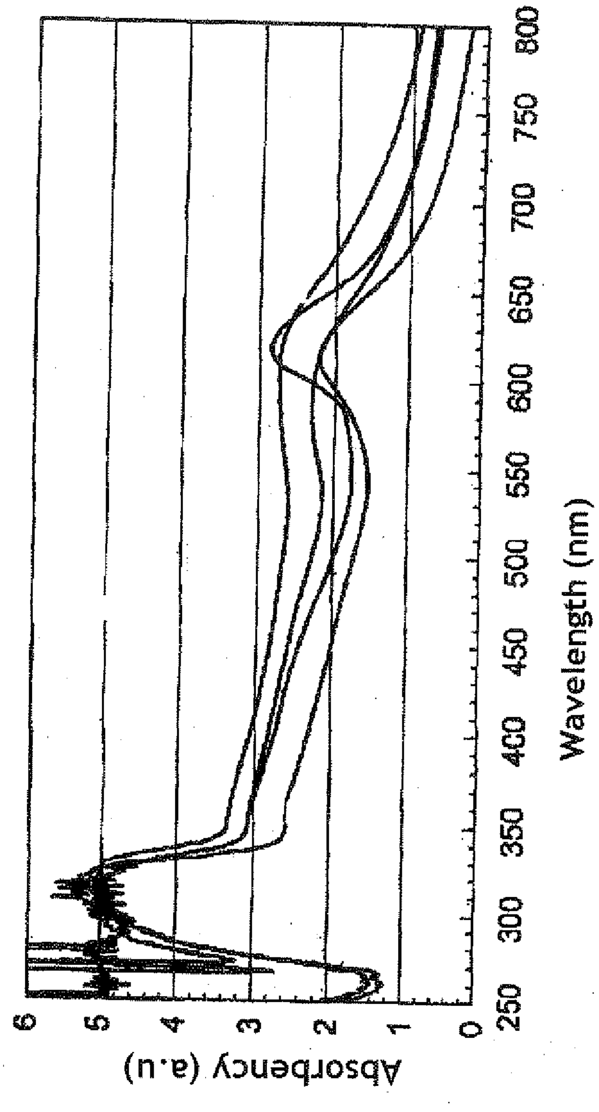

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 06136724 A
- WO 08007267 A
- US 6894086 B
- US 20030116062 A
- US 20060002875 A
- US 20030148088 A
- FR 2833487
- FR 2848428
- FR 2910286
- WO 2007082061 A2
- WO 04055081 A
- EP 1411069 A
- WO 04028488 A
- FR 0302809
- US 5624663 A
- US 5237071 A
- US 5166355 A
- GB 2303549 A
- DE 19726184
- EP 0893119 A
- EP 0832642 A
- EP 1027883 A
- EP 1300137 A
- DE 0162844
- WO 9304665 A
- DE 19855649
- EP 0967200 A
- DE 19746654
- DE 19755649
- EP 1008586 A
- EP 1133980 A
- EP 0133981 A
- WO 04006878 A
- WO 05058269 A
- WO 06032741 A
- US 6225467 B
- WO 2004085412 A
- WO 06035000 A
- WO 06034982 A
- WO 06034991 A
- WO 06035007 A
- WO 2006034992 A
- WO 2006034985 A
- EP 0669323 A
- US 2463264 A


### Non-patent literature cited in the description


- *J. Wang, Polym Int,* 2008, vol. 57, 509-514
- **A. STEIN.** *Chem Mater,* 2002, vol. 14, 3305-3315
- **E. THOMAS.** *Nat Mat,* 2008, vol. 6, 957-960
- A new substrate to measure sunscreen protection factors throughout the ultraviolet spectrum. *J. Soc. Cosmet. Chem.,* 1989, vol. 40, 127-133
- **S-H KIM et al.** *JACS,* 2006, vol. 128, 10897-10904
- **S-M YANG.** *Langmuir,* 2005, vol. 21, 10416-10421
- **LI et al.** *J. Mater. Chem.,* 2005, vol. 15, 2551-2556
- **D. PINE.** *Langmuir,* 2005, vol. 21, 6669-6674
- **S-H KIM et al.** *Adv Mater,* 2008, vol. 9999, 1-7
- **LI et al.** Ordered macroporous titania photonic balls by micrometer-scale spherical assembly templating. *J. Mater. Chem.,* 2005, vol. 15, 2551-2556
- **D. PINE ; F. LANGE.** *Langmuir,* 2005, vol. 21, 6669-6674
- **A. STEIN.** *Chem. Mater.,* 2002, vol. 14, 3305-3315
- **D. PINE ; F. F. LANGE.** *Langmuir,* 2005, vol. 21 (15), 6669-6674
- **F. F. LANGE.** *Colloid Polym Sci,* 2003, vol. 282, 7-13
- **WANG et al.** Effects of fabrication conditions on the characteristics of etamidazole spray dried microspheres. *J. Microencapsulation,* 2002, vol. 19 (4), 495-510
- **S-H KIM et al.** *Adv. Mater.,* 2008, vol. 9999, 1-7
- **KIM et al.** *Adv. Mater.,* 2008, vol. 20, 3211-3217
- Symmetrical Triazine Derivatives. IP.COM Journal. IP.COM INC, 20 September 2004
- *Cosmetics & Toiletries,* February 1990, vol. 105, 53-64
- CTFA Cosmetic Ingredient Handbook. Cosmetic and Fragrance Assn, Inc, 2004